# EUROPÄISCHE PATENTANMELDUNG

(11) **EP 2 524 709 A1**
(43) Veröffentlichungstag der Anmeldung: **21.11.2012**
(21) Anmeldenummer: 11075087.4
(22) Anmeldetag: 16.05.2011
(51) Int. Cl.: A61M 1/10, A61F 2/06, A61B 17/11

(54) **Verbindungssystem zum lösbaren Fixieren eines hohlzylindrischen Bauteils an einer Ausnehmung**

(71) Anmelder: Berlin Heart GmbH, 12247 Berlin (DE)
(72) Erfinder: Göllner, Manfred, 13086 Berlin (DE); Marcinowski, Daniel, 12619 Berlin (DE)
(74) Vertreter: Pfenning, Meinig & Partner GbR

(57) **Zusammenfassung**

Zur Herstellung einer leicht lösbaren Verbindung eines hohlzylindrischen Bauteils (9), zum Beispiel eines Grafts, an einer Ausnehmung (2) in einem Gewebe beispielsweise eines Herzens oder eines Blutgefäßes sind ein Ringkörper (13), ein Führungskörper (15) und ein Federkörper (19) vorgesehen, die über einen Nahtring (4) einerseits an dem Körperteil und andererseits an dem hohlzylindrischen Bauteil befestigt sind und die eine leicht lösbare Verriegelung gegeneinander mittels des Federkörpers erlauben. Der Ringkörper weist hierzu eine an seinem Umfang umlaufende Nut (14) auf, in die der Federkörper zur Verriegelung eintaucht.

## Beschreibung

Die Erfindung liegt auf dem Gebiet der Mechanik, speziell der Verbindungstechnik, und bezieht sich noch spezieller auf die Herstellung einer Verbindung mit hohlzylindrischen Bauteilen. Besonders vorteilhaft ist die Erfindung auf dem Gebiet der Medizintechnik einsetzbar.

In der medizinischen Technik sind häufig, insbesondere bei Operationen, bei denen der Blutkreislauf betroffen ist, hohlzylindrische Bauteile in Form von Kanülen, Grafts, Rohrstutzen und Ähnlichem miteinander oder mit Teilen eines Patientenkörpers wie beispielsweise Blutgefäßen oder Organen zu verbinden.

Ein typischer Anwendungsfall ist die Verbindung eines Grafts mit einem Loch in einer Herzwand oder einem großen Blutgefäß, wobei zunächst ein Nahtring mittels einer Nahtverbindung mit dem organischen Gewebe verbunden und daraufhin entsprechende hohlzylindrische Bauteile mit dem Nahtring verbunden werden.

Entsprechende hohlzylindrische Bauelemente können auch teilweise in die Öffnung des betroffenen Körperteils wie beispielsweise in die Herzwand oder in die Öffnung eines Blutgefäßes wenigstens ein Stück weit hineinragen.

Es sind aus dem Stand der Technik verschiedene Varianten der Verbindung von hohlzylindrischen Bauelementen mit der Öffnung eines Körperteils eines Patientenkörpers bekannt. Beispielsweise zeigt die US 2007/0134993 A1 einen Nahtring, der nach Art eines Flansches mit Körpergewebe verbunden werden kann, wobei mit dem Nahtring ein Rohrstutzen fest verbunden ist. Der Rohrstutzen weist einen Längsschlitz auf, der mittels einer Schraube überbrückt ist, so dass der Stutzen durch Anziehen der Schraube radial auf einem eingesteckten Rohrstück festklemmbar ist.

Eine sehr ähnliche Konfiguration zeigt auch die US 2007/0167969 A1. Dort liegt der Schwerpunkt allerdings auf einem Werkzeug zum gezielten Einbringen einer Öffnung in ein Körperteil.

Aus der WO 2007/084340 A2 (dort Fig. 1 in Zusammenhang mit Textseite 6) ist ebenfalls ein Nahtring mit einem an diesem befestigten Klemmring bekannt, wobei der Klemmring radial durch eine an seinem Umfang befindliche Schraube komprimiert werden kann.

Da zum Anziehen der im Stand der Technik beschriebenen Schrauben einerseits bei einer Operation möglicherweise nicht vorhandener Platz benötigt wird, andererseits ein solches Festziehen auch Zeit benötigt und das Herstellen einer derartigen Klemmverbindung allgemein auch schlecht überprüfbar ist, hat sich die vorliegende Erfindung zur Aufgabe gestellt, ein Verbindungssystem für derartige Fälle zu schaffen, das ein einfaches und lösbares Verbinden eines hohlzylindrischen Bauteils mit einer Ausnehmung erlaubt und das mit möglichst einfachen konstruktiven Mitteln verwirklichbar ist.

Die Aufgabe wird mit den Mitteln der Erfindung gemäß den Merkmalen des Patentanspruchs 1 gelöst.

Bei dem Verbindungssystem zum lösbaren Fixieren eines hohlzylindrischen Bauteils an einer Ausnehmung in einem Teil eines Patientenkörpers ist demnach gemäß der Erfindung ein Ringkörper vorgesehen, der an seiner äußeren Mantelfläche eine sich wenigstens abschnittsweise azimutal erstreckende Nut aufweist, sowie ein Führungskörper, der vor einer Fixierung gegenüber dem Ringkörper in Axialrichtung des zylindrischen Bauteils verschiebbar ist. Der Führungskörper und der Ringkörper können wenigstens teilweise in Axialrichtung ineinanderschiebbar sein, so dass sie einander in Axialrichtung teilweise überlappen. Zudem ist ein Federkörper vorgesehen, der in dem Führungskörper ausschließlich senkrecht zur Axialrichtung beweglich geführt ist und wenigstens teilweise in die Nut des Ringkörpers eintaucht. Damit fixiert der Federkörper den Ringkörper einerseits und den Führungskörper andererseits gegeneinander in Axialrichtung. Zur Fixierung des hohlzylindrischen Bauteils an der Ausnehmung ist entweder der Ringkörper oder der Führungskörper mit dem hohlzylindrischen Bauteil verbunden oder gekoppelt und der jeweils andere der beiden Körper mit dem Rand der Ausnehmung verbunden oder gekoppelt.

Auf diese Weise lässt sich das hohlzylindrische Bauteil über den Ringkörper und den Führungskörper und den diese verriegelnden Federkörper an der Ausnehmung lösbar befestigen.

Der Ringkörper kann beispielsweise zylindrisch aufgebaut sein, und die an seinem Umfang umlaufende Nut kann auch als vollständige Ringnut auf seinem gesamten Umfang umlaufen. Die Ringnut kann im Querschnitt rechteckig, V-förmig oder U-förmig ausgebildet sein. Über eine Anschrägung der Wände der Ringnut kann es auch ermöglicht werden, beim Auftreten übermäßiger axialer Kräfte das hohlzylindrische Bauteil abzuziehen, indem der Federkörper aus der Nut gedrängt und die Verbindung zwischen dem Ringkörper und dem Führungskörper gewaltsam gelöst wird.

Der Führungskörper kann eine zylindrische Bohrung für das hohlzylindrische Bauteil und zusätzlich einen Hohlraum zur Führung des Federkörpers aufweisen. Hierzu kann der Führungskörper im Wesentlichen aus zwei plattenartigen und miteinander verbindbaren Teilkörpern bestehen.

Der Federkörper kann entweder steif oder federnd ausgebildet sein, muss jedoch so dimensioniert werden, dass er als Feder in die Nut des Ringkörpers wenigstens teilweise eintauchen kann.

Jedes der Bauteile, also sowohl der Ringkörper als auch der Führungskörper und der Federkörper, kann aus einem Metall, vorzugsweise eine Titanlegierung, oder aber aus Kunststoff, beispielsweise aus einem harten Silikon, einem Polyethylen oder einem Polyurethan bestehen. Es können auch ein oder mehrere Bauteile aus einem ersten dieser Werkstoffe und ein anderes der Bauteile aus einem anderen Material bestehen.

Der Ringkörper und der Führungskörper sind jeweils entweder mit dem hohlzylindrischen Bauteil oder mit dem Rand der Ausnehmung verbunden oder gekoppelt. Dies bedeutet, dass die jeweiligen Bauteile mittelbar oder unmittelbar mit dem hohlzylindrischen Bauteil und dem Rand der Ausnehmung verbunden sind. Es kann also jeweils noch ein weiteres Bauteil vorgesehen sein, das seinerseits mit dem hohlzylindrischen Bauteil oder mit dem Rand der Ausnehmung verbunden ist und mit dem jeweils der Ringkörper oder der Führungskörper verbunden oder gekoppelt ist. Der Ringkörper und der Führungskörper können bei der Verbindung mit dem hohlzylindrischen Bauteil beispielsweise auf diesem begrenzt in Axialrichtung verschiebbar sein, wobei die Begrenzung der Verschiebbarkeit beispielsweise durch einen an dem Bauteil befestigt Anschlag realisiert sein kann.

Es kann vorteilhaft bei der Erfindung vorgesehen sein, dass der Federkörper in dem Führungskörper in sich unverformt verschiebbar geführt ist. Dabei weist der Federkörper innerhalb des Führungskörpers wenigstens eine erste Stellung auf, in der er nach dem Ineinanderschieben von Führungskörper und Ringkörper in die Nut des Ringkörpers eintaucht, sowie wenigstens eine zweite Position, in der der Federkörper in die Nut des Ringkörpers nicht eintaucht. Zwischen diesen Positionen ist der Federkörper schwenkbar, drehbar oder verschiebbar.

Beispielsweise kann der Ringkörper im Querschnitt in seiner äußeren Kontur elliptisch ausgebildet sein, und der Federkörper kann eine entsprechend elliptische Öffnung aufweisen, in die der Ringkörper in einer ersten Orientierung hineinpasst. Werden der Ringkörper und der Federkörper dann gegeneinander um die Achse des hohlzylindrischen Bauteils gedreht, so kann der Federkörper in die Ringnut des Ringkörpers eintauchen und die Verriegelung bewirken. Der Federkörper weist zu diesem Zweck einen Betätigungshebel auf, der aus dem Führungskörper herausragt und von außen zum Drehen des Federkörpers betätigbar ist.

Es kann auch vorteilhaft vorgesehen sein, dass der Federkörper innerhalb des Führungskörpers zwischen einem Verriegelungszustand und einem unverriegelten Zustand, in dem er nicht in die Nut des Ringkörpers eintaucht, elastisch verformbar ist. Auch in diesem Fall ist der Federkörper vorteilhaft so gebaut, dass er wenigstens teilweise derart aus dem Führungskörper herausragt, dass die elastische Verformung von außen manuell bewirkt werden kann.

Eine weitere vorteilhafte Ausgestaltung der Erfindung kann vorsehen, dass der Federkörper elastisch federnde Bestandteile aus einem Kunststoff oder einem Metall aufweist, insbesondere ganz aus einem derartigen federnden Material besteht.

Eine weitere Ausgestaltung der Erfindung sieht vor, dass der Federkörper elastisch radial in Bezug auf die Axialrichtung des zylindrischen Körpers aufweitbar ist.

Durch die radiale Verformbarkeit ist der Federkörper vorteilhaft in einer ersten Radialrichtung komprimierbar und senkrecht dazu gleichzeitig radial aufweitbar. Auf diese Weise kann er durch radialen Druck elastisch derart verformt werden, dass er in einer ersten Form über den Ringkörper hinübergeschoben werden kann und in einer zweiten Form in die Nut des Ringkörpers wenigstens teilweise eintaucht.

Die Ausdehnung des ringförmigen Federkörpers kann dazu in einer ersten Richtung größer sein als in einer quer zur ersten Richtung verlaufenden zweiten Richtung.

Der Federkörper kann beispielsweise als elliptisch geformter geschlossener Drahtbügel oder als im Wesentlichen rautenförmiger oder hexagonal geformter Drahtbügel ausgeführt sein. Durch radialen Druck auf zwei einander gegenüberliegende Seiten kann der Federkörper in diesem Fall insgesamt aufgeweitet bzw. zusammengedrückt werden.

Der Ringkörper muss für eine sinnvolle Verwendung mit einem solchen Federkörper im Querschnitt derart geformt sein, dass der Federkörper über den Ringkörper in einer ersten Form herübergeschoben werden kann und in einer zweiten Form in die Nut des Ringkörpers wenigstens teilweise eintaucht. Hierzu kann beispielsweise vorgesehen sein, dass der Durchmesser eines dem ringförmigen Federkörper einbeschriebenen Kreises im entspannten Zustand kleiner ist als der Außendurchmesser des Ringkörpers.

Eine vorteilhafte Ausgestaltung der Erfindung sieht zudem vor, dass der mit dem Rand der Ausnehmung verbundene oder verbindbare Körper mit einem unmittelbar mit dem Rand verbundenen oder verbindbaren Befestigungskörper verbunden oder verbindbar ist. Auf diese Weise kann ein entsprechender Befestigungskörper vor dem Anbringen des Verbindungssystems mit dem Rand der Ausnehmung beispielsweise durch Nähen oder andere übliche Verbindungstechniken verbunden werden. Oft handelt es sich bei einem derartigen Befestigungskörper um einen filz- oder gewebeartig ausgeführten Nahtring, der über eine Fadenverbindung mit einem Gewebe des Patientenkörpers verbindbar ist. Der Ringkörper oder der Führungskörper können mit dem Befestigungskörper bereits vor dessen Fixierung an dem Gewebe bzw. am Rand der Ausnehmung fest verbunden sein. Sie können jedoch mit dem Befestigungskörper über eine Klemm-, Kleb- oder Schraubverbindung auch nach der Befestigung am Rand der Ausnehmung verbunden werden.

Eine weitere vorteilhafte Ausgestaltung der Erfindung sieht vor, dass der Befestigungskörper ein Nahtring ist, der an organischem Gewebe am Rand der Ausnehmung durch eine Nahtverbindung mittels eines Fadens befestigbar ist.

Es kann zudem vorgesehen sein, dass der Befestigungskörper eine umlaufende Dichtlippe, beispielsweise aus Silikon, aufweist, an der das diese durchsetzende hohlzylindrische Bauteil dichtet. Die Dichtlippe kann an einer Öffnung des Befestigungskörpers vorgesehen sein und dort in das Innere der Öffnung hineinragen. In diesem Fall dichtet die Dichtlippe des Befestigungskörpers außen an der zylindrischen Mantelfläche des hohlzylindrischen Bauteils. Es muss dann nur noch die mechanische Fixierung des hohlzylindrischen Bauteils bewerkstelligt werden, wobei die Dichtigkeit der Verbindung ausschließlich durch die Dichtlippe gewährleistet werden kann.

Im Folgenden wird die Erfindung anhand eines Ausführungsbeispiels in einer Zeichnung gezeigt und anschließend beschrieben.

Dabei zeigt
- Fig. 1: in einem Schnitt schematisch eine Herzwand mit einem daran befestigten Nahtring,
- Fig. 2: die Anordnung aus Fig. 1 mit einem eingeschobenen Rohrstutzen,
- Fig. 3: eine alternative Befestigungsart eines Rohrstutzens in einer Öffnung in einer Herzwand,
- Fig. 4: schematisch in einem Längsschnitt ein Verbindungssystem gemäß der Erfindung im nicht fixierten Zustand,
- Fig. 5: die Anordnung aus Fig. 4 im fixierten Zustand,
- Fig. 6: einen Teil eines Führungskörpers,
- Fig. 7: den Teil des Führungskörpers aus Fig. 6 mit einem eingelegten Federkörper,
- Fig. 8: den kompletten Führungskörper mit einem Federkörper,
- Fig. 9: eine Draufsicht auf einen Schnitt des Führungskörpers mit einem Federkörper im Verriegelungszustand,
- Fig. 10: einen Schnitt wie in Fig. 9, jedoch mit einem elastisch verformten und entriegelten Federkörper,
- Fig. 11: zwei Teile eines Führungskörpers in einer dreidimensionalen Explosionsdarstellung,
- Fig. 12: einen alternativen Führungskörper mit einem lediglich verschiebbaren Federkörper sowie
- Fig. 13: einen weiteren alternativen Führungskörper, ebenfalls mit einem verschiebbaren Federkörper.

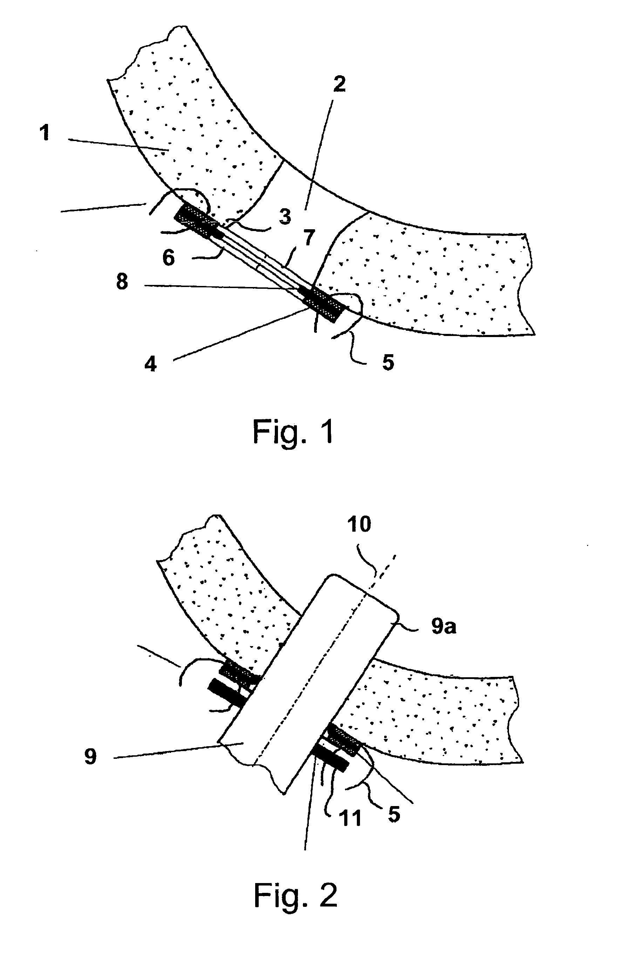

**Figur 1** zeigt schematisch einen Teil einer Herzwand 1 mit einer durchgehenden Ausnehmung 2, die beispielsweise auch künstlich durch Stanzen oder Schneiden in die Herzwand eingebracht sein kann.

Auf den Rand 3 der Ausnehmung 2 ist ein Nahtring 4 aufgelegt, der mittels einer umlaufenden chirurgischen Naht durch einen Faden 5 mit dem organischen Gewebe der Herzwand 1 verbunden werden kann. Ein solcher Nahtring kann beispielsweise aus einem filzartigen Gewebe bestehen, das mittels einer Nähnadel einfach durchstoßen werden kann. In dem gezeigten Ausführungsbeispiel weist der Nahtring 4 eine erste Gewebeschicht 6, eine zweite Gewebeschicht 7 und eine dazwischen liegende Dichtschicht 8 mit einer radial nach innen vorspringenden Dichtlippe aus Silikon auf.

Der Nahtring 4 dient dazu, eine zuverlässige Verbindung eines Leitungs- oder Rohrelementes an der Herzwand 1 zu ermöglichen und zu erleichtern. Das Leitungselement kann ein Pumpenanschluss, gegebenenfalls Teil eines Pumpengehäuses sein.

**Figur 2** zeigt einen in die Ausnehmung 2 eingeschobenen zylindrischen Gegenstand, der typischerweise als Leitungselement bzw. als hohlzylindrischer Graft 9 ausgebildet ist. Dieser dient dazu, einen Leitungskanal vom Inneren des Herzens oder auch eines anderen Organs zu einem gewünschten anderen Punkt herzustellen. Ein Graft ist üblicherweise flexibel, er kann jedoch auch ein steifes Ende aufweisen, das in eine Ausnehmung 2 einschiebbar ist. Das Ende 9a sollte jedenfalls in der Ausnehmung frei um seine Längsachse 10 drehbar sein. Das Ende 9a des Grafts weist einen Befestigungsflansch 11 auf, der beispielsweise ebenfalls aus einem Gewebe bestehen oder ein Gewebe enthalten kann und der mit dem Nahtring 4 vernäht werden kann, um das Ende des Grafts in der Ausnehmung 2 an dem Nahtring zu fixieren.

Der Flansch kann auch aus einem harten Material, insbesondere einem einem Metall oder einem harten Kunststoff, bestehen und Öffnungen aufweisen, die das Durchtreten des Fadens 5 zur Fixierung an dem Nahtring erlauben.

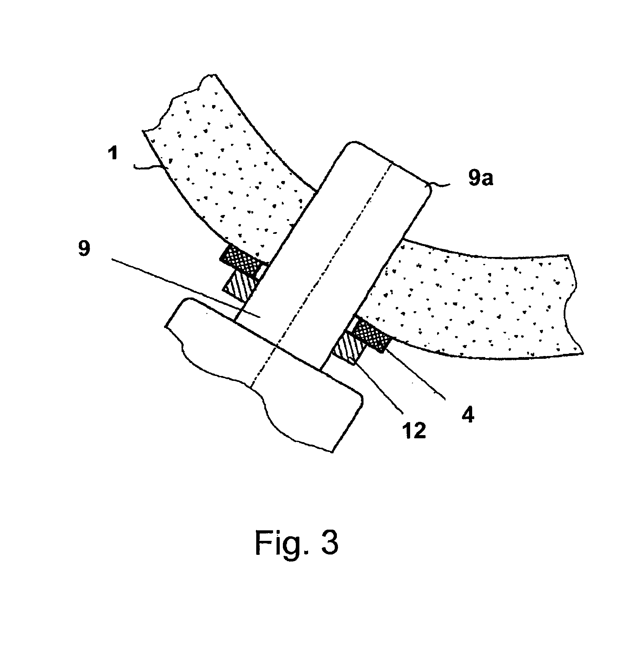

**Figur 3** zeigt eine andere Befestigungsart, bei der mit dem Nahtring 4 direkt und unmittelbar ein Klemmring 12 fest verbunden ist, der in Längsrichtung geschlitzt und somit radial aufweitbar ist, um das Ende 9 des Grafts problemlos einschieben zu können. Nach dem Positionieren des Endes 9a des Grafts wird der Klemmring 12 mittels einer nicht im Einzelnen dargestellten Klemmschraube oder Schelle angezogen, d.h. auch radial komprimiert, um das Ende 9a zu fixieren und fest zu positionieren. Dabei kann der Klemmring 12 so dimensioniert sein, dass er gleichzeitig auch dichtet.

Jedoch ist die Betätigung eines Klemmmechanismus für einen derartigen Klemmring 12 einerseits unter Operationsbedingungen aufwendig, und andererseits wäre auch ein einfaches Lösen dieser Verbindung wünschenswert.

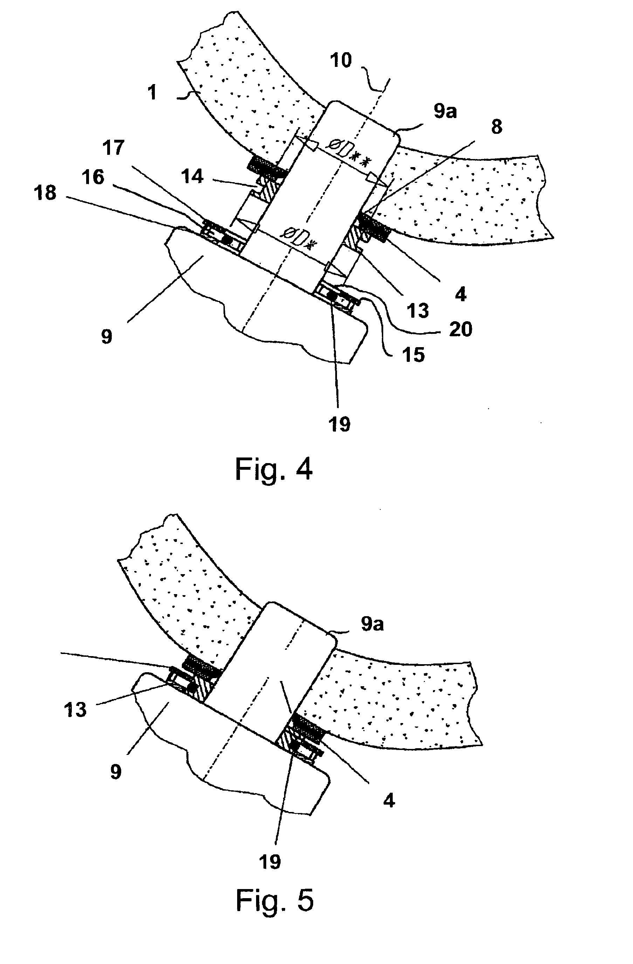

**Figur 4** stellt eine erfindungsgemäße Ausgestaltung eines Verbindungssystems dar, mit dem das Ende 9 eines hohlzylindrischen Bauteils in einer Ausnehmung einer Herzwand 1 lösbar befestigbar ist, wobei die Verbindung ebenso leicht herstellbar wie wieder lösbar ist.

Zu diesem Zweck ist in der Darstellung der Fig. 4 ein mit der Herzwand 1 verbundener Nahtring 4 vorgesehen, mit dem ein Ringkörper 13 fest verbunden ist, der eine an seiner äußeren Mantelfläche azimutal umlaufende Nut 14 aufweist. Die Nut 14 ist im Querschnitt reckeckig ausgebildet, sie könnte jedoch auch V-förmig oder rund gestaltet sein.

Weiter ist ein Führungskörper 15 vorgesehen, der mit dem hohlzylindrischen Bauteil 9 fest verbunden ist und der in einem Hohlraum 16 zwischen zwei Frontplatten 17, 18 einen Federkörper 19 führt.

Die Frontplatte 17 weist eine zentrische Ausnehmung 20 auf, die so groß ist, dass der Führungskörper 15 auf den Ringkörper 13 wenigstens teilweise axial aufgeschoben werden kann.

Zum Aufschieben des Führungskörpers 15 in axialer Richtung der Zylinderachse 10 auf den Ringkörper 13 wird der Federkörper 19 derart verformt, dass er ebenfalls über den Ringkörper 13 geschoben werden kann und elastisch in die Nut 14 radial eintaucht. Der Federkörper kann einfach durch manuelle Krafteinwirkung oder durch Auflaufen auf eine Fase am Ringkörper verformt werden. Da der Federkörper 19 weiterhin in dem Führungskörper 15 gehalten ist, kann er somit den Ringkörper 13 und den Führungskörper 15 gegeneinander in axialer Richtung der Achse 10 verriegeln.

**Figur 5** zeigt detailliert den Verbindungszustand in verriegelter Form, bei dem der Federkörper 19 bereits in der Nut 14 des Ringkörpers 13 eingerastet ist. Das Ende 9 des hohlzylindrischen Körpers ist somit in der Ausnehmung der Herzwand 1 mittels des Nahtrings 4 leicht lösbar fixiert. Grundsätzlich kann anstelle der Fixierung eines Ringkörpers 13 am Nahtring und der Befestigung des Führungskörpers 15 an dem hohlzylindrischen Körper auch umgekehrt der Ringkörper 13 mit dem Führungskörper vertauscht und seinerseits mit dem hohlzylindrischen Körper verbunden sein, während der Führungskörper 15 in diesem Fall mit dem Nahtring 4 oder in anderer Weise direkt mit dem Rand der Ausnehmung 2 in der Herzwand 1 verbunden ist.

Das Verbindungssystem kann im Übrigen auch bei anderen Organen oder beispielsweise bei Blutgefäßen angewendet werden, wobei dann die Herzwand 1 durch die Wand eines Blutgefäßes ersetzt ist.

Der hohlzylindrische Körper 9 kann typischerweise aus einem Metall, insbesondere einer Titanlegierung, jedoch auch aus einem Kunststoff, beispielsweise auch aus einem Wellrohr, bestehen, wobei er in dem Bereich, in dem er in den Nahtring einschiebbar ist, durchgehend zylindrisch mit gleichbleibendem Durchmesser ausgeführt sein kann, um eine Dichtung der Dichtlippe 8 dort zu erlauben. Dieses Ende 9a des Körpers/Grafts kann beispielsweise aus einem anderen Material als der übrige Bereich hergestellt sein, beispielsweise aus PTFE, Polyurethan oder einem Metall.

Der hohlzylindrische Körper kann beispielsweise als Anschlussstutzen einer Pumpe, insbesondere einer Herzpumpe, oder als Pumpenrohr, welches gleichzeitig einen Teil des Pumpengehäuses bildet, ausgebildet sein.

Der Führungskörper 15 sowie der Ringkörper 13 können vorzugsweise aus Metall, insbesondere einer Titanlegierung, beispielsweise derselben Legierung wie der hohlzylindrische Körper 9, bestehen, jedoch auch in besonderen Fällen aus einem widerstandsfähigen Kunststoff wie Polyurethan, aus PTFE oder Polyethylen. Der Federkörper 19 kann ebenfalls aus einem der genannten Materialien bestehen, jedoch muss dieser elastisch verformbar sein. Der Federkörper 19 kann als Metalldraht mit entsprechend elastischen Eigenschaften ausgeführt sein.

Der Federkörper 19 sollte zudem so geformt sein, dass er aus einer umfangsseitig an dem Führungskörper 15 vorgesehenen Öffnung derart herausragt, dass er von dort bewegbar bzw. komprimierbar ist.

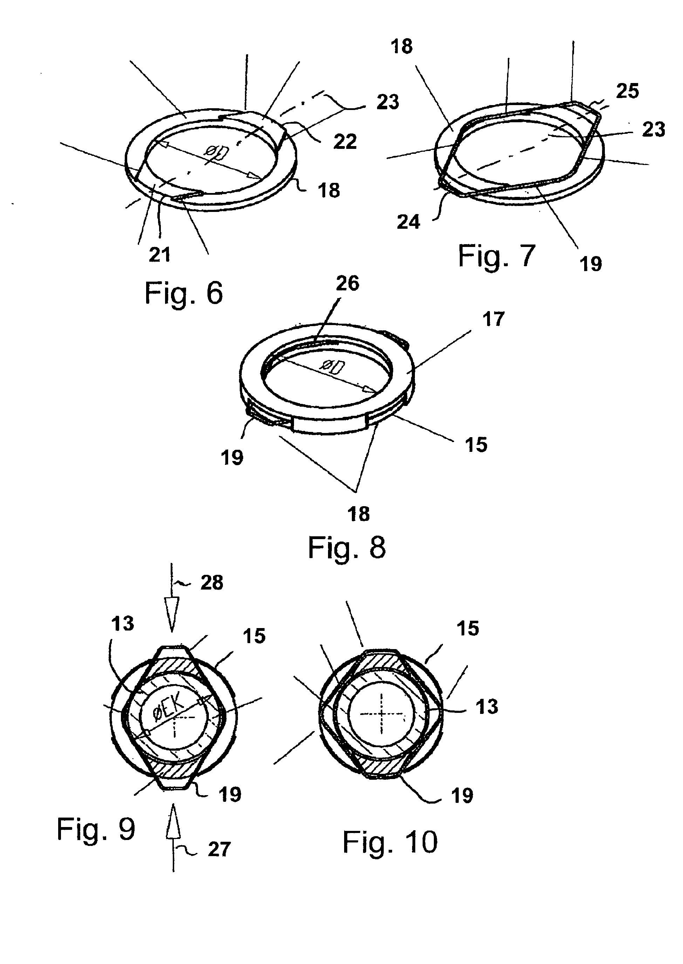

**Figur 6** zeigt in einer dreidimensionalen Darstellung eine Frontplatte 18 des Führungskörpers, die beispielsweise aus einem Kunststoff besteht, im Wesentlichen kreisringförmig ausgebildet ist und zwei Verdickungen 21, 22 aufweist, die einander symmetrisch auf dem Ring bezüglich einer Achse 23 gegenüberliegen, die ihrerseits eine Symmetrieachse der Frontplatte 18 darstellt. Die Höhe der Verdickungen 21, 22 kann beispielsweise etwa der Dicke des Federkörpers 19 entsprechen oder etwas größer sein als diese.

**Figur 7** zeigt die Frontplatte 18 aus Fig. 6 mit einem aufgelegten Federkörper 19, der hier durch einen geschlossenen Drahtbügel in rautenähnlicher Form gebildet ist. Die Rautenform des Federkörpers 19 weist lediglich zwei Abstumpfungen 24, 25 auf, die der besseren Greifbarkeit im zusammengebauten Zustand dienen. Der Federbügel ist durch die Verdickungen 21, 22 gehalten und derart geführt, dass er ausschließlich in Richtung der Achse 23 komprimierbar und quer zur Achse 23 damit radial aufweitbar ist. Die abgestumpften Enden 24, 25 können zu diesem Zweck mit zwei Fingern ergriffen und aufeinander zubewegt werden, um den Federkörper 19 aufzuweiten.

**Figur 8** zeigt den Führungskörper 15 in zusammengebauter Form mit der ersten Frontplatte 17 und der zweiten Frontplatte 18. Die Frontplatten 17, 18 sind so geformt, dass sie zwischen sich im zusammengebauten Zustand den Raum für den Federkörper 19 frei lassen. Insbesondere kann die erste Frontplatte 17 auf den Verdickungen 21, 22 aufliegen. Zwischen den Frontplatten 17, 18 ist in der Fig. 8 der Federkörper 19 dargestellt, der aus Öffnungen an der äußeren Mantelfläche des Führungskörpers herausragt und dort manuell betätigbar ist. An der Stelle 26 ist sichtbar, dass der Federkörper 19 im entspannten Zustand in die lichte zentrische Öffnung des Führungskörpers hineinragt. Mit dieser lichten Öffnung kann der Führungskörper 15 allerdings, wenn der Federkörper 19 betätigt ist, auf den Ringkörper 13 aufgeschoben werden. Nach dem Aufschieben kann der Federkörper 19 losgelassen werden, so dass er radial nach innen in die Nut 14 des Ringkörpers einschnappt.

**Figur 9** zeigt in einer senkrechten Draufsicht einen Schnitt des Führungskörpers 15 mit einem unbetätigten Federkörper 19. Durch zwei Pfeile 27, 28 sind die Betätigungskräfte des Federkörpers bezeichnet. Zusätzlich zu dem Führungskörper 15 ist in dessen Innerem auch der Ringkörper 13 eingezeichnet, wobei der Federkörper 19 in der Draufsicht den Ringkörper 13 teilweise durchdringt, da er in dessen umlaufende Nut eingetaucht ist.

**Figur 10** zeigt dagegen den gleichen Schnitt wie Fig. 9 mit einem betätigten Federkörper 19, der durch die Kraftwirkung so weit aufgeweitet ist, dass er radial an keiner Stelle den Umfang des Ringkörpers 13 berührt. In dieser Stellung kann der Führungskörper 15 mit dem Federkörper 19 auf den Ringkörper 13 aufgeschoben und von diesem abgezogen werden.

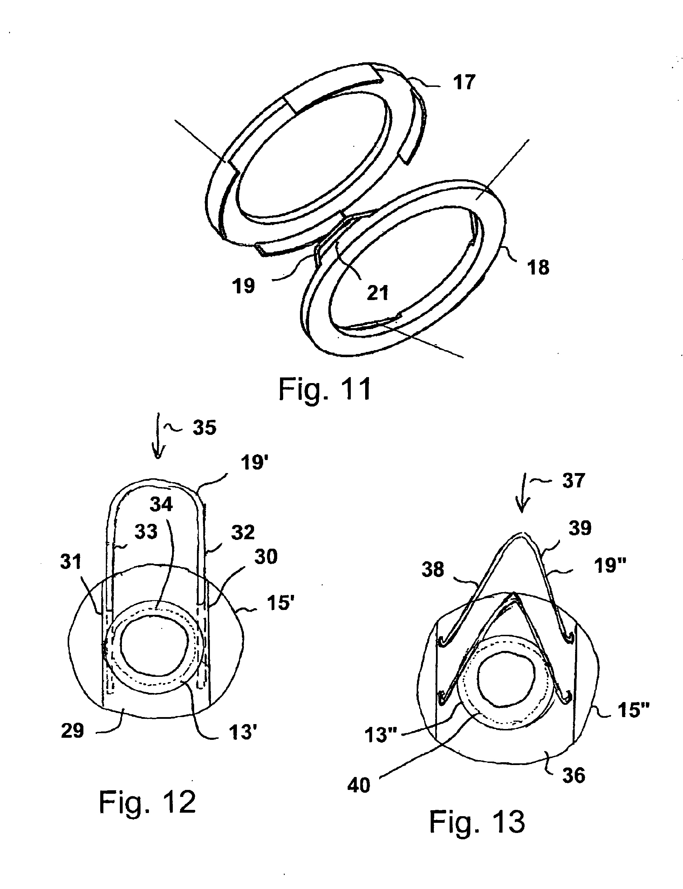

**Figur 11** zeigt schematisch das Zusammenwirken der Frontplatten 17, 18, bevor diese zu einem Führungskörper zusammengesetzt sind. An der Frontplatte 18 sind eine Verdickung 21 sowie der eingelegte Federkörper 19 zu erkennen.

**Figur 12** zeigt eine alternative Ausgestaltung eines Führungskörpers, eines Ringkörpers und eines Federkörpers. Der Führungskörper 15' ist mit einem darin konzentrisch befindlichen Ringkörper 13' dargestellt. Der Führungskörper 15' weist eine weite Nut 29 oder auch nur zwei Führungskanäle 30, 31 für die zwei Schenkel 32, 33 des Federkörpers 19' auf. Der Federkörper muss nicht aufgeweitet werden, um in die Nut des Ringkörpers 13' eingeschoben zu werden, die durch den gestrichelten Grund 34 mit der Nut dargestellt ist. Der Federkörper 19' kann einfach in Richtung des Pfeils 35 radial in den Führungskörper 15' eingeschoben werden, um in die Nut des Ringkörpers einzutauchen und Feder und Führungskörper gegeneinander zu verriegeln. Die Führung des Federkörpers 19' im Führungskörper 15' kann so straff ausgeführt werden, dass der Federkörper sich im Führungskörper selbsttätig verklemmt. Es können jedoch auch die Schenkel 32, 33 des Federkörpers 19' so lang ausgebildet werden, dass sie durch den Führungskörper hindurchstoßen und auf der anderen Seite durch einen Sicherungsring gesichert werden können.

**Figur 13** zeigt eine weitere Ausgestaltung eines Führungskörpers sowie eines Federkörpers mit einem Führungskörper 15", der eine Nut zur Aufnahme des V-förmigen Federkörpers 19'' aufweist. Der Federkörper kann beispielsweise in der Nut 36 des Führungskörpers 15" zwischen den Nutwänden verklemmt sein. Dabei ist der Federkörper in Richtung des Pfeils 37 in die Nut 36 mindestens so weit einschiebbar, bis die Schenkel 38, 39 in die Nut des Ringkörpers 13" eintauchen, die durch den gestrichelt dargestellten Grund 40 der Nut repräsentiert ist. Dadurch, dass der Federkörper 19" sich in der Nut 36 des Führungskörpers 15" selbsttätig verklemmt, ist er auch im verriegelten Zustand selbsttätig fixiert.

Die Beispiele aus den Figuren 12 und 13 zeigen, dass die Verriegelung zwischen dem Führungskörper und dem Ringkörper auch ohne einen in sich verformbaren Federkörper nur mittels eines verschieb-, dreh- oder schwenkbaren Federkörpers realisiert werden kann.

Die dargestellten Implementierungen der Erfindung erlauben jedenfalls ein einfaches und lösbares Verbinden eines hohlzylindrischen Bauteils 9 an einer Öffnung 2 im Gewebe eines Patientenkörpers, wobei die Verbindung einfach auch unter Operationsbedingungen herstellbar und flüssigkeitsdicht ist. Das hohlzylindrische Bauteil ist dabei, gegebenenfalls zusammen mit daran befestigten weiteren Elementen wie Pumpen oder Ventilen, leicht in der Öffnung drehbar.

## Patentansprüche

1. Verbindungssystem zum lösbaren Fixieren eines hohlzylindrischen Bauteils (9) an einer Ausnehmung (2) in einem Teil eines Patientenkörpers mit einem Ringkörper (13; 13'; 13''), der an seiner äußeren Mantelfläche eine sich zumindest abschnittsweise azimutal erstreckende Nut (14) aufweist, und mit einem Führungskörper (15; 15'; 15"), der vor einer Fixierung gegenüber dem Ringkörper in Axialrichtung (10) des hohlzylindrischen Bauteils (9) verschiebbar ist, wobei der Führungskörper (15; 15'; 15") und der Ringkörper wenigstens teilweise in Axialrichtung ineinanderschiebbar sind, sowie mit einem Federkörper (19; 19'; 19''), der in dem Führungskörper ausschließlich senkrecht zur Axialrichtung (10) beweglich derart geführt ist, dass er zur Herstellung des fixierten Zustandes wenigstens teilweise in die Nut (14) des Ringkörpers eintaucht und damit den Ringkörper und den Führungskörper gegeneinander in Axialrichtung verriegelt, wobei im Verbindungszustand von dem Ringkörper und dem Führungskörper jeweils einer mit dem hohlzylindrischen Bauteil (9) und der andere Körper zur unmittelbaren oder mittels eines Verbindungselementes (4) hergestellten Verbindung oder Kopplung mit dem Rand (3) der Ausnehmung (2) vorgesehen ist.

2. Verbindungssystem nach Anspruch 1, **dadurch gekennzeichnet, dass** der Federkörper (19'; 19") in dem Führungskörper (15'; 15") in sich unverformt verschiebbar geführt ist.

3. Verbindungssystem nach Anspruch 1 oder 2,
**dadurch gekennzeichnet, dass** der Federkörper (19) innerhalb des Führungskörpers (15) zwischen einem Verriegelungszustand und einem unverriegelten Zustand, in dem er nicht in die Nut (14) des Ringkörpers (13) eintaucht, elastisch verformbar ist.

4. Verbindungssystem nach Anspruch 1 oder einem der folgenden, **dadurch gekennzeichnet, dass** der Federkörper (19) elastisch federnde Bestandteile aus einem Kunststoff oder einem Metall aufweist, insbesondere ganz aus einem derartigen federnden Material besteht.

5. Verbindungssystem nach Anspruch 1 oder einem der folgenden, **dadurch gekennzeichnet, dass** der Federkörper (19) elastisch radial in Bezug auf die Axialrichtung des zylindrischen Körpers aufweitbar ist.

6. Verbindungssystem nach Anspruch 1 oder einem der folgenden, **dadurch gekennzeichnet, dass** der Federkörper (19) ein ringförmig geschlossener strangförmiger Körper ist.

7. Verbindungssystem nach Anspruch 6, **dadurch gekennzeichnet, dass** die Ausdehnung des ringförmigen Federkörpers (19) in einer ersten Richtung größer ist als in einer quer zur ersten Richtung verlaufenden zweiten Richtung.

8. Verbindungssystem nach Anspruch 6 oder 7,
**dadurch gekennzeichnet, dass** der ringförmige Federkörper (19) durch radial zur Ringmitte gerichteten Druck quer zur Druckrichtung aufweitbar ist.

9. Verbindungssystem nach Anspruch 1 oder einem der folgenden, **dadurch gekennzeichnet, dass** der Durchmesser eines dem ringförmigen Federkörper (19) einbeschriebenen Kreises im entspannten Zustand kleiner ist als der Außendurchmesser des Ringkörpers (13).

10. Verbindungssystem nach Anspruch 1 oder einem der folgenden, **dadurch gekennzeichnet, dass** der mit dem Rand (3) der Ausnehmung (2) verbundene oder verbindbare Körper (13; 13'; 13"; 15; 15'; 15") mit einem unmittelbar mit dem Rand verbundenen oder verbindbaren Befestigungskörper (4) verbunden oder verbindbar ist.

11. Verbindungssystem nach Anspruch 10, **dadurch gekennzeichnet, dass** der Befestigungskörper (4) ein Nahtring ist, der an organischem Gewebe am Rand (3) der Ausnehmung (2) durch eine Nahtverbindung mittels eines Fadens befestigbar ist.

12. Verbindungssystem nach Anspruch 10 oder 11,
**dadurch gekennzeichnet, dass** der Befestigungskörper (4) eine umlaufende Dichtlippe (8) aufweist, an der das diese durchsetzende hohlzylindrische Bauteil (9) dichtet.
